# EUROPEAN PATENT APPLICATION

(11) **EP 1 667 082 A1**
(43) Date of publication of application: **07.06.2006**
(21) Application number: 04787774.1
(22) Date of filing: 09.09.2004
(51) Int. Cl.: G08C 17/02, G01K 1/02, H01Q 1/36, H01Q 1/38, H04B 1/034

(54) **RADIO MODULE, RADIO TEMPERATURE SENSOR, RADIO INTERFACE DEVICE, AND RADIO SENSOR SYSTEM**

(30) Priority: 11.09.2003 JP 2003319357; 19.09.2003 JP 2003328846; 29.09.2003 JP 2003338219; 13.07.2004 JP 2004206297; 03.08.2004 JP 2004226773
(71) Applicant: MITSUBISHI MATERIALS CORPORATION, Chiyoda-ku, Tokyo 100-8117 (JP)
(72) Inventor: YOKOSHIMA, Takao, Tokyo 112-0002 (JP); NAKAMURA, Kenzo, Tokyo 112-0002 (JP); TARI, Kazuyoshi, Tokyo 112-0002 (JP); NAGIRA, Tsumoru, Tokyo 112-0002 (JP); KAMIJYO, Hiroki, 12-14, Koishikawa 1-chome,, Dept Tokyo 1120002 (JP); KISHI, Yasunari, 12-14, Koishikawa 1-chome,, Dept Tokyo 1120002 (JP)
(74) Representative: HOFFMANN EITLE
(86) International application number: PCT/JP2004/013110
(87) International publication number: WO 2005/027073

(57) **Abstract**

The invention provides a wireless temperature sensor that is attached to the patient's skin in order to measure the patient's temperature, thereby adopting a patient moving in the facilities as a measuring object.

The wireless sensor is a wireless temperature sensor having a function of wirelessly transmitting measured data through a chip antenna, and the chip antenna has a length of a shortening coefficient so as to be received in a container, and is received in the container.

In addition, the invention provides a wireless interface device in which a wireless communication function is provided to a CompactFlash (registered trade mark) card having a low carrier frequency, a small antenna, an enlarged communication distance, and low power consumption.

Furthermore, the invention provides a wireless sensor system in which the sensor notifies a patient of surrounding environment information by a wireless communication device whereby a base station collects the environment information from a plurality of sensors.

## Description

### BACKGROUND OF THE INVENTION

### 1. Field of the Invention

The present invention relates to a wireless module, a wireless temperature sensor that measures temperature of an object to be contacted and transmits a measured result by means of wireless communication, a wireless interface device used in an external interface such as a PDA (Personal Digital Assistant) and PC (Personal Computer), and a wireless sensor system in which a sensor notifies a patient of surrounding environment information by a wireless communication device whereby a base station collects the environment information from a plurality of sensors.

### 2. Description of the Related Art

Since it is necessary to check the patient's temperature around the clock in a hospital or nursing-care facility, a medical staff directly measures the patient's temperature by means of a clinical thermometer.

However, in the above-mentioned method of measuring the patient's temperature, since it is necessary to periodically measure temperatures of many patients of which the medical staff takes care, it takes the medical staff all times thereof to measure temperature of many patients. Accordingly, there has been a problem that the medical staff lacks time.

Accordingly, a temperature sensor for directly reading measured data by means of a terminal, for example, a temperature sensor that is buried in the body and measures temperature by using a resonant frequency has been developed in order to simply measure the patient's temperature (For example, see JP-A-62-192137).

However, since the temperature sensor disclosed in JP-A-62-192137 should be buried in the body of a patient, the use thereof is limited.

Since it is not necessary that the temperature sensor be buried in the body of a general patient, the medical staff should come to see the patients in order to measure temperature when measuring temperature of many patients. Accordingly, there has still been a problem.

In addition, a method, which measures the patient's temperature by connecting the clinical thermometer to a computer, has been proposed. However, it is not possible to use the method in a case in which the patient moves in the facilities.

Furthermore, recently, a local area communication, for example, a communication in one indoor area is performed by means of an IC card serving as an interface of a PC card standard (based on the standard according to PCMIA) among a PDA, various information processing systems, and the like (for example, see JP-A-2001-195553).

The IC card is used as a recording medium for data, and is also used for expansion of peripheral devices.

However, as described above, the wireless interface disclosed in JP-A-2001-195553 is formed in the shape of an IC card.

Accordingly, the wireless interface does not correspond to an interface (slot or expansion slot) of an external memory, which is used for expansion of the memory, such as a CF (CompactFlash (registered trade mark)), SD card (registered trade mark), SmartMedia (registered trade mark) or the like, which has been recently used as a standard of the PDA.

In addition, frequencies of 2.4 and 5 GHz, which are used in the wireless LAN or the like, are used in the interface of the IC card as carrier frequencies. However, there have been problems in that a sufficient communication distance cannot be obtained by means of the above-mentioned frequencies and the frequencies are suitable for a mobile terminal requiring large power consumption such as a PDA.

Meanwhile, when a faint radio frequency or predetermined small power radio frequency is used, a long communication distance can be obtained and low power consumption is required, however, the carrier frequency thereof is low unlike in the wireless LAN. Accordingly, the wavelength thereof is long and a long antenna is required, whereby a degree of freedom of the PDA is reduced and the appearance of the interface deteriorates.

Furthermore, when measuring the environment information, for example, temperature of a measuring object, the conventional wireless sensor calculates temperature from the resistance values (measured values of sensors) measured by sensor devices, and transmits the temperature (environment information) as calculation results to a base station (see JP-A-2-138837).

For this purpose, the conventional wireless sensor compensates the environment information (for example, temperature) for each sensor device, and then transmits the environment information to the base station, thereby obtaining accurate numerals of the environment information.

However, in the wireless sensor according to the related art, many operations are performed to convert physical quantities, that is, measured values (resistance value, voltage value, current value, pressure value, frequency value, and the like) of the sensor into environment information (temperature, humidity, snow cover, concentration of various gases, and the like). Accordingly, there is a problem that the power consumption thereof is increased.

Furthermore, in the wireless sensor according to the related art, the structure of the wireless sensor becomes complicated to convert physical quantities, that is, measured values of the sensor into numerals of the environment information. Accordingly, the price of the wireless sensor rises.

In order to cope with the above-mentioned problems, a wireless sensor according to the related art directly transmits the measured values measured by the sensor devices to the base station, and performs an operation for converting the physical quantities, that is, measured values into numerals of the environment information in the base station. As a result, the processing loads of the sensor devices are reduced and the structure of the wireless sensor becomes simple.

Meanwhile, in the method, since the sensor has only one function, that is, an operation processing function corresponding to a predetermined sensor device, the system of the sensor has a low degree of freedom. Accordingly, there has been a problem that the structure of the system has a low degree of freedom in configuring another system by replacing the sensor device to the other sensor device or several kinds of sensor devices.

In addition, when the above-mentioned sensor system uses a look-up table method to convert the physical quantities, that is, measured values into the environment information, the above-mentioned sensor system uses the same conversion table. Accordingly, it is difficult to configure a system by using several kinds of sensor devices

Furthermore, the above-mentioned sensor system converts the physical quantities, that is, measured values (live data measured by the sensor) of the sensor into only numerals of the environment information, and does not perform a correction of the measured values and a correction of converted environment information. In addition, the above-mentioned sensor system cannot cope with the secular change of each sensor.

### SUMMARY OF THE INVENTION

The invention has been made to solve the above-mentioned problems, and it is an object of the invention to provide a wireless temperature sensor that is attached to patient's skin in order to measure the patient's temperature, thereby adopting a patient moving in the facilities as a measuring object. Furthermore, it is another object of the invention to provide a wireless interface device in which a wireless communication function is provided to a CompactFlash (registered trade mark) card having a low carrier frequency, a small antenna, an enlarged communication distance, and low power consumption. In addition, it is still another object of the invention to provide a wireless sensor system that transmits measured values of sensor devices and corrects the measured values and converted physical quantities, thereby obtaining accurate environment information.

According to an aspect of the invention, a wireless temperature sensor is sealed in a container of a sensor main body and has a function of wirelessly transmitting measured data through an antenna. The antenna has a length of a shortening coefficient so as to be received in the container, and is received in the container.

The above-mentioned wireless temperature sensor further includes a matching circuit for adjusting the impedance between the antenna and a line for a transmission signal.

In the above-mentioned wireless temperature sensor, the antenna and an electric circuit including the matching circuit are formed on one substrate, and the antenna is mounted on the substrate in an area where a grounding wire is not formed.

In the above-mentioned wireless temperature sensor, the substrate is positioned in the container so that the substrate is separated from a contact surface to be attached to a measuring object with a predetermined distance therebetween.

In the above-mentioned wireless temperature sensor, on the substrate, a grounding point of a high frequency circuit is connected to a grounding point of a logic circuit through a filter for blocking a signal of a carrier frequency band used for communication.

In the above-mentioned wireless temperature sensor, the container has the same size as a 500-yen coin.

In the above-mentioned wireless temperature sensor, the container is formed in the shape of a coin that has a diameter of 9 to 27 mm and a thickness of 5 to 10 mm.

In the above-mentioned wireless temperature sensor, a length of the antenna is shorter than an eighth of the wavelength of an electric wave having a frequency to be used.

In the above-mentioned wireless temperature sensor, the frequency to be used is in the range of 300 to 960 MHz.

In the above-mentioned wireless temperature sensor, the antenna includes an antenna substrate, a conductor film formed on a part of the antenna substrate, a feeding point formed on the antenna substrate, a loading part that is mounted on the antenna substrate and includes a linear-conductor pattern formed on the surface of an element made of a dielectric material in the longitudinal direction of the element, an inductor part for connecting one end of the conductor pattern with the conductor film, and a feeding point used to feed electric power to a node between the one end of the conductor pattern and the inductor part. Further, the antenna is mounted on the substrate so that the longitudinal direction of the loading part is parallel to one side of the conductor film.

According to another aspect of the invention, a wireless interface device corresponds to an interface of a memory and includes an antenna having a wireless communication function. The antenna, which is used to transmit and receive a carrier wave, has a length of a shortening coefficient so as to be received in the memory having a standard dimension. The antenna is mounted in the wireless interface device.

The above-mentioned wireless interface device further includes a matching circuit for adjusting the impedance between the antenna and a line for a transmission signal.

In the above-mentioned wireless interface device, a grounding point of a high frequency circuit is connected to a grounding point of a logic circuit through a filter for blocking a signal of a carrier frequency band used for communication .

In the above-mentioned wireless interface device, the antenna includes a substrate, a conductor film formed on a part of the substrate, a feeding point formed on the substrate, a loading part that is mounted on the substrate and includes a linear-conductor pattern formed on the surface of an element made of a dielectric material in the longitudinal direction of the element, an inductor part for connecting one end of the conductor pattern with the conductor film, and a feeding point used to feed electric power to a node between the one end of the conductor pattern and the inductor part. Further, the antenna is mounted in the wireless interface device so that the longitudinal direction of the loading part is parallel to one side of the conductor film.

In a wireless sensor system according to the invention, the above-mentioned wireless temperature sensor further includes a plurality of wireless sensors that is provided at several positions, and that obtains measured values corresponding to surrounding environment information by means of a sensor device and transmits the values; a data-collecting terminal that is provided to a base station, and that receives the measured values from the wireless sensors and calculates environment information from the measured values to collect the environment information of the several positions; a sensor for outputting the surrounding environment information as measured values based on the physical property of the sensor device; and a wireless transmitting part (a wireless transmitting / receiving part 2, a wireless transmitting part 2B) for wirelessly transmitting measured data including the measured values and identification data. Furthermore, the data-collecting terminal includes a conversion information memory, which stores conversion information used to convert a measured value of the sensor device of each registered wireless sensor into environment information, and a converter, which determines the wireless sensor by means of the identification data and converts the measured value into environment information by means of physical property and conversion information corresponding to each wireless sensor.

In a wireless sensor system according to the invention, the above-mentioned wireless temperature sensor further includes a memory in which the physical property and conversion information of the sensor device is stored. Furthermore, during the registration for the data-collecting terminal, the wireless terminal transmits the physical property and conversion information to the data-collecting terminal, and the data-collecting terminal stores the physical property and conversion information in the conversion information memory in correspondence with the identification number of the wireless sensor.

In a wireless sensor system according to the invention, the conversion information includes initial deviation of the physical property and compensation information of the physical property.

In a wireless sensor system according to the invention, the correction coefficient is stored in the sensor device used in the wireless sensor as data of the annual change of the physical property, which is statistically obtained, and a time cycle of correction and the correction coefficient are transmitted to the data-collecting terminal at the time of registration.

In a wireless sensor system according to the invention, the wireless sensor measures an output voltage of a battery for supplying a driving electric power at a predetermined time cycle, and transmits information for directing battery replacement to the data-collecting terminal when the output voltage is smaller than a predetermined voltage.

### BRIEF DESCRIPTION OF THE DRAWINGS

Fig. 1 is a conceptual diagram showing a front mounting surface of a substrate 10 of a wireless temperature sensor 100 according to an embodiment of a first aspect of the invention;
Fig. 2 is a conceptual diagram showing a rear mounting surface of the substrate 10 of the wireless temperature sensor 100 according to the embodiment;
Fig. 3 is a perspective cross-sectional view showing the wireless temperature sensor 100 according to the embodiment;
Fig. 4 is a conceptual diagram showing an example of circuits of the wireless temperature sensor 100 according to the embodiment;
Fig. 5 is a block diagram showing an exemplary structure of a matching circuit 8;
Fig. 6 is a graph showing the relationship between reflection power and control voltage;
Fig. 7 is a conceptual diagram showing a structure in which ground wires of a high frequency circuit 17 and a digital circuit 18 are connected to each other through a band reject filter 19;
Fig. 8 is a plan view showing a chip antenna 1a according to another embodiment;
Fig. 9 is a perspective view showing the chip antenna 1a according to the embodiment;
Fig. 10 is a graph showing a frequency characteristic of a VSWR of the chip antenna 1a according to the embodiment;
Fig. 11 is a graph showing the radiation pattern of the chip antenna 1a according to the embodiment;
Fig. 12 is a schematic view showing a structure 10 of a wireless interface device 2-1 according to a first embodiment of a second aspect of the invention;
Fig. 13 is a circuit diagram showing an exemplary structure of an impedance control circuit that is provided in the wireless interface device 2-1;
Fig. 14 is a graph showing the relationship between reflection power and control voltage of the impedance control circuit;
Fig. 15 is a circuit diagram showing a structure of a GND wire on a substrate that is provided in the wireless interface device 2-1;
Fig. 16 is a plan view showing a wireless interface device 2-10 according to a second embodiment of a second aspect of the invention;
Fig. 17 is a perspective view showing the wireless interface device 2-10 according to the embodiment;
Fig. 18 is a graph showing a frequency characteristic of a VSWR of the wireless interface device 2-10 according to the embodiment;
Fig. 19 is a graph showing the radiation pattern of the wireless interface device 2-10 according to the embodiment;
Fig. 20 is a view showing a shape of an USB connector 2-40 which is provided with the wireless interface device 2-10 according to the embodiment;
Fig. 21 is a block diagram showing an exemplary structure of a wireless sensor system according to an embodiment of a third aspect of the invention;
Fig. 22 is a block diagram showing an exemplary structure of a temperature sensor, which is an example of a sensor device 3-3 shown in Fig. 21; and
Fig. 23 is a sequence diagram showing a flow of a registration process in a data collection terminal 3-7 of the wireless sensor system 3-1 according to the embodiment.

### DESCRIPTION OF THE PREFERRED EMBODIMENTS

### <First aspect>

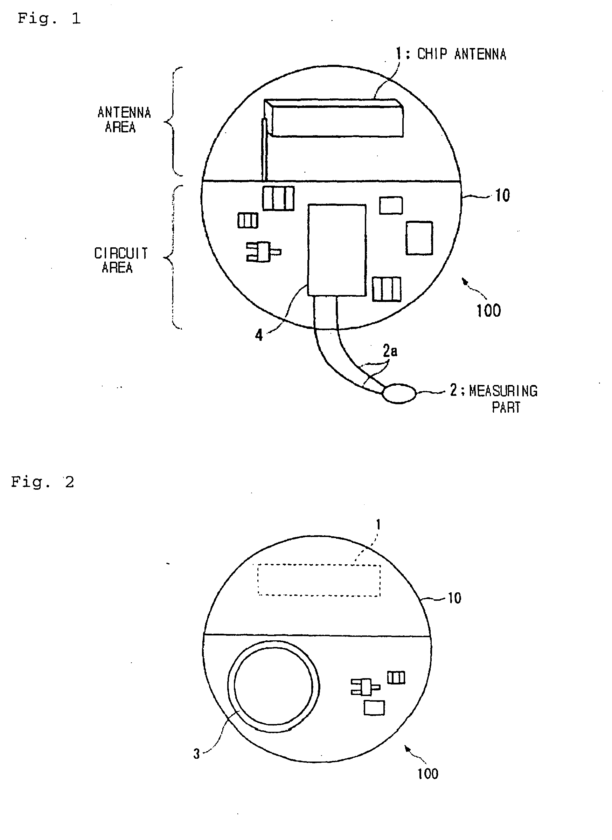

Hereinafter, a wireless temperature sensor 100 according to an embodiment of the invention will be described with reference to Figs. 1 to 11. Fig. 1 is a conceptual diagram showing a structure of a front mounting surface of a substrate 10 on which components of the wireless temperature sensor 100 according to the embodiment are mounted. In addition, Fig. 2 is a conceptual diagram showing a structure in which components are mounted on a rear mounting surface of the substrate 10.

Hereinafter, a wireless temperature sensor 100, which is attached to (or comes in close contact with) a patient's skin in order to measure the patient's temperature, will be described as an example of the embodiment of the invention.

As shown in Figs. 1 and 2, a chip antenna 1 is positioned in an area, which is distant from the periphery of the substrate 10 by a predetermined distance, on the mounting surface of the substrate 10. Furthermore, the chip antenna is mounted on the substrate in an area where a grounding wire (GND wire) is not formed. In addition, an electronic component such as an IC, which forms a circuit of the wireless temperature sensor 100, is mounted in a circuit area where a grounding wire is formed.

For this reason, it is possible to considerably reduce the ratio of the capacitive coupling between the chip antenna 10 and the grounding wire. Accordingly, it is possible to improve the efficiency of the energy for communication by reducing a coupling loss and limiting needless energy consumption.

In addition, a measuring part 2 is a resistance element (such as a NTC thermistor or the like) of the sensor for directly measuring temperatures, and is connected to the sensor unit 4 by connecting lines 2a. A battery 3 is mounted on the rear surface of the wireless temperature sensor 100 (see Fig. 2) in order to supply driving electric power to each circuit of the wireless temperature sensor 100.

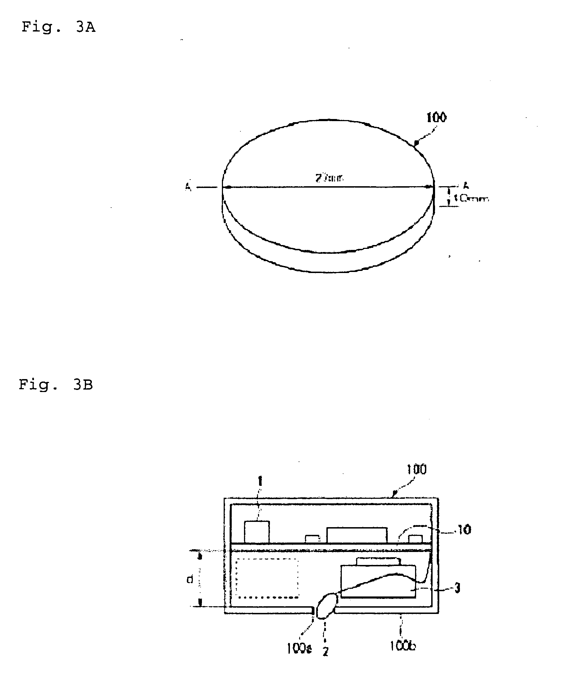

Fig. 3A shows a size and shape of the wireless temperature sensor 100 (container). The wireless temperature sensor has a shape similar to that of a 500-yen coin, and the chip antenna 1 or the substrate 10 is received in a coin-shaped container that has a diameter of about 9 to 27 mm and a thickness of about 5 to 10 mm (see Fig. 1).

Fig. 3B is a cross-sectional view, which shows the wireless temperature sensor 100, taken along line A of Fig. 3A.

As shown in Fig. 3B, the substrate 10 of the wireless temperature sensor 100, on which the circuits and the chip antenna 1 are mounted,.is positioned in the container so that the substrate is separated from a contact surface 100b to be attached to (or come in close contact with) a measuring surface of a measuring object (for example, a patient) with a predetermined distance d therebetween. That is, the substrate is positioned in the container so that the chip antenna 1 and the measuring object are separated from each other with a predetermined distance therebetween.

Since the predetermined distance d is formed, it is possible to considerably reduce the capacitive coupling between the chip antenna 1 and the measuring object. Accordingly, it is possible to considerably reduce the energy for transmission.

Furthermore, the measuring part 2 is fixedly disposed to the container so that a unit of the measuring part protrudes by a predetermined distance outward through an opening 100a formed on the contact surface 100b.

As a result, since the measuring part 2 directly comes in close contact with the patient's skin, it is not necessary to wait until the entire container has the same temperature as the patient and the measuring part can accurately measure the patient's temperature. Accordingly, it is possible to cope with the rapid variation of the patient's temperature, and to accurately detect the temperature variation.

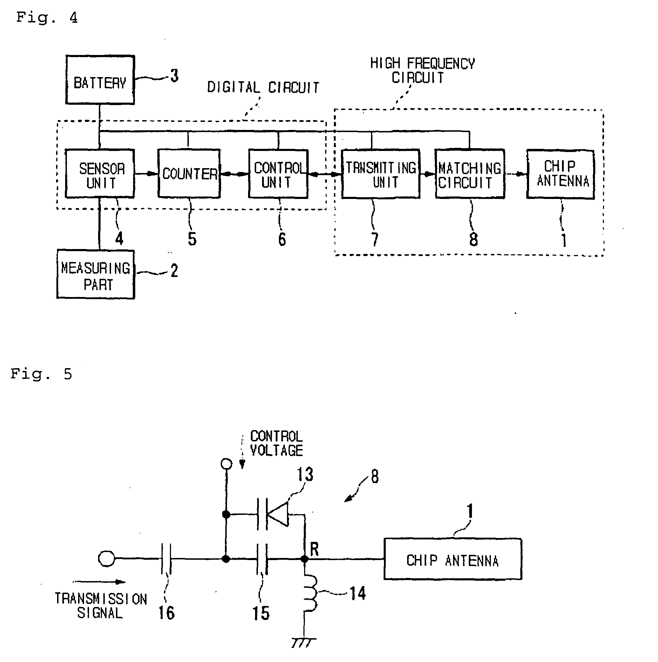

Next, the circuits formed on the substrate 10 will be described with reference to Fig. 4. Fig. 4 is a block diagram showing an exemplary structure of circuits of the wireless temperature sensor 100.

An oscillator, which includes a Wien bridge circuit stable against voltage variations and temperature variations, is provided in the sensor unit 4.

The oscillator determines an oscillation frequency depending on the resistance value of the measuring part 2 including a thermistor and the like. As the resistance value of the measuring part 2 is varied depending on the temperature variations, the oscillation frequency is varied so as to correspond to the temperature variations. For this reason, even though there are voltage variations of the battery 3, it is possible to detect the resistance variations of the measuring part 2 depending on temperature variations by using the oscillation frequency. Therefore, it is possible to reliably measure temperatures.

When the above-mentioned measured data is received by a receiving unit, the receiving unit extracts an identification number and an oscillation frequency, thereby reading out the temperatures corresponding to the oscillation frequency from a table showing the relationship between oscillation frequency and temperature. Then, the read temperatures are time-sequentially stored in a database as temperature data for every identification number.

In addition, predetermined thresholds of an upper limit and a limit of the oscillation frequency may be set in a control unit of the wireless temperature sensor 100. For example, when it is determined that the measured oscillation frequency is not in the range of an upper limit threshold to a lower limit threshold, that is, it is detected that the patient's temperature is not in the normal range, the sensor may further have a function for giving notice to the patient or a medical staff by ringing a buzzer.

A counter 5 counts the number of oscillation pulses of the oscillator provided in the sensor 4, and transmits the counted value to a transmitting unit 7 in every period (for example, thirty minutes). Then, after the counter resets the counted value to be 'zero', the counter newly counts the number of oscillation pulses during a predetermined period.

That is, the sensor unit 4 outputs the patient's temperature, which is a measuring object, to the counter 5 in the form of the oscillation frequency based on the resistance value (physical quantity) of the measuring part 2 (such as a NTC (Negative Temperature Coefficient) thermistor or the like).

The counter 5 measures the pulses output from the sensor unit 4 in the form of the counted value (which represents an oscillation frequency) during a predetermined period, and outputs the counted value as a measured result to the control unit 6.

The control unit 6 adds the identification number of the wireless temperature sensor 100 to the counted value, and outputs the counted value having the identification number as measured data to the transmitting unit 7.

The transmitting unit 7 modulates a carrier wave by using the measured data, and outputs the modulated carrier wave serving as a RF signal of a transmission signal to the matching circuit 8.

In addition, when the chip antenna 1 is received in the container, which has a diameter of 27 mm and is formed in the shape of a 500-yen coin, the length of the chip antenna 1 is limited to about 27 mm. Accordingly, when a faint radio frequency or predetermined small power radio frequency of 300, 400, 900, or 960 MHz band is used as a frequency of the carrier wave to be used, the chip antenna 1 needs to have the following length due to the fact that the length of an antenna is a quarter of a wavelength.

| Frequency | a quarter of a wavelength |
|---|---|
| 300 MHz | 250 mm |
| 400 MHz | 188 mm |
| 900 MHz | 83 mm |
| 960 MHz | 78 mm |

Therefore, in the present embodiment, shortening coefficients of 89% or more, 85% or more, 67% or more, and 65% or more are used to design / manufacture an antenna for frequencies of 300, 400, 900, and 960 MHz bands, respectively, so that the chip antenna 1 is received in the container of the wireless temperature sensor 100 having a diameter of 27 mm.

In the above-mentioned chip antenna 1, a resonant circuit for transmitting and receiving an electric wave is a composed of a resonant circuit, which includes an inductance component and a capacitance component, in order to configure an antenna having a high shortening coefficient and a small antenna having a high gain by using a circuit.

Furthermore, the chip antenna 1 according to the present embodiment includes a plurality of resonant circuits to obtain a high gain. In addition, two or more resonant circuits, in which the inductance component and the capacitance component are electrically connected in parallel with each other, are electrically connected in series with each other.

Moreover, the inductance component includes a coil, which is made of a conductor and is formed in a spiral shape or in the shape of a substantial spiral polygon about an axis thereof. The axis of the coil is provided at least in the resonant areas adjacent to each other in a shape of a substantial straight line, and at least one of parts of the conductor going around the axis is substantially included in the plane inclined with respect to the axis.

According to the wireless temperature sensor 100 of the present embodiment, when the wireless communication is performed, a faint radio frequency or predetermined small power radio frequency in the range of 300 to 960 MHz band is used as a frequency of the carrier wave without requiring strict use examination. Accordingly, it is possible to enlarge a communication distance, and to reduce power consumption.

In addition, in the present embodiment, the wireless temperature sensor 100, which uses a frequency of the carrier wave having a long wavelength, has been described. In this case, shortening coefficients are calculated and used to design the antenna so that the chip antenna 1 is received in the container of the wireless temperature sensor 100 having the same size and shape as those of a 500-yen coin, that is, so that the chip antenna 1 has a length and a width to be received in the container of the wireless temperature sensor 100.

However, since the chip antenna 1 includes the inductance component and the capacitance component, the chip antenna is easy to be affected by surroundings. That is, when the wireless temperature sensor 100 is shipped, there is a case that the impedance of the chip antenna matching with the impedance of a line for a transmission signal is varied by the influence of a metal case such as a container of the wireless temperature sensor 100, and the radiation characteristic thereof deteriorates. In order to remove the influence of the metal case, it is preferable that the chip antenna 1 be disposed away from the metal case of the wireless temperature sensor 100. However, this departs from the original object in which the chip antenna 1 having a large shortening coefficient is mounted in the wireless temperature sensor 100.

Accordingly, the wireless temperature sensor 100 according to the present embodiment is provided with the matching circuit 8 shown in Fig. 5 for adjusting the impedance.

In the matching circuit 8, a variable capacitance diode 13 and a capacitor 15 are connected in parallel with each other, and a DC blocking capacitor 16 is connected to one end of the variable capacitance diode and the capacitor connected in parallel with each other. Then, a coil 14 is connected to the other end thereof. Furthermore, the chip antenna 1 is connected to a node R between the variable capacitance diode 13 and the capacitor 15.

When the transmission signal (a RF signal that is a traveling wave) input from the transmitting unit 7 is reflected at a contact point (node R) having different impedance, the traveling wave is affected by a reflected wave. Accordingly, a composite wave, in which the traveling wave and the reflected wave are combined, is generated on the line. The composite wave is referred to as a standing wave, and a ratio between the maximum voltage | Vmax | of the standing wave and the minimum voltage | Vmin | thereof is referred to as a voltage standing wave ratio (VSWR). When the reflection does not occur, the VSWR is 1. That is, as the value of the VSWR is reduced, the reflection is reduced. In case of a small antenna such as the chip antenna 1, a reference VSWR of the node R is about 3 or less.

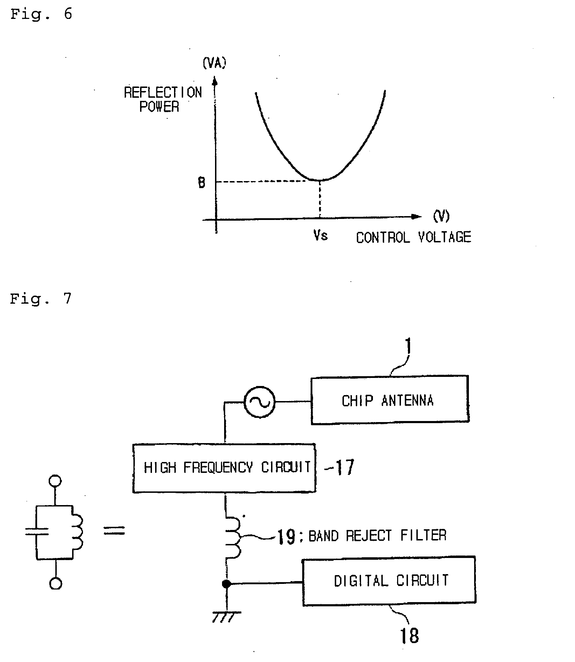

The control unit 6 measures the VSWR of the node R, and applies control voltage to the matching circuit so that the VSWR is about 3 or less. The variable capacitance diode 13 has a characteristic in which the capacitance thereof is varied due to the applied control voltage (reverse voltage), thereby adjusting the impedance of the line. The relationship between the reflection power and the control voltage is shown in Fig. 6. A horizontal axis of Fig. 6 represents the control voltage (V), and a vertical axis thereof represents the reflection power (VA).

Therefore, according to the wireless temperature sensor 100 of the present embodiment, in a state in which the matching circuit is mounted in the wireless temperature sensor 100, the matching circuit 8 adjusts impedance between the chip antenna 1 and the line for the transmission signal. As a result, it is always possible to transmit the transmission signal so that transmission power has an almost maximum value. Accordingly, it is possible to prevent the radiation characteristic from deteriorating, and to improve receiver sensitivity.

When the wireless temperature sensor 100 having the chip antenna 1 therein is driven, high frequency current caused by a digital signal of a digital circuit is input to a high frequency circuit through the GND (ground) wire.

As a result, since a noise caused by the high frequency current is superimposed on the transmission signal, there is a possibility that the transmission wave having a noise component (radiation noise) is radiated from the chip antenna 1.

Specifically, if the frequency of the noise is in a carrier frequency band or in the vicinity of the carrier frequency band, the noise is radiated as a strong radiation noise. Accordingly, the noise has a negative effect on the reception characteristic of other wireless devices, which use the same carrier frequency band as the frequency of the noise.

However, since the frequency of the radiation noise is different every device, it is difficult to remove the radiation noise from the receiving unit.

For this reason, as shown in Fig. 7, the wireless temperature sensor 100 of the present embodiment modulates the carrier wave by using a modulation wave so as to generate the transmission signal. In addition, the GND wire of a high frequency circuit 17, to which a signal is radiated from the chip antenna 1, and the GND wire of a digital circuit 18 for processing the measured data are connected to each other through a band reject filter 19 (or band elimination filter). The band reject filter blocks an only wave of a predetermined frequency band (a carrier frequency band used to transmit and receive the data by the wireless sensor or a frequency range including the vicinity of the carrier frequency band).

That is, the GND wire of the high frequency circuit 17 is connected to the GND wire of the wireless temperature sensor 100 through the band reject filter 19. Accordingly, the high frequency current of the carrier frequency band and of the vicinity of the carrier frequency band, which is generated by the digital circuit 18, is not input to the GND wire of the high frequency circuit 17 as a noise, and it is possible to prevent the radiation noise from being generated.

Furthermore, the substrate 10 provided in the wireless temperature sensor 100 is configured so as to reduce the spatial coupling (capacitive coupling) between the GND wire of the digital circuit 18 and the GND wire of the high frequency circuit 17. Accordingly, the GND wires are not formed on the upper and lower surfaces of the substrate 10, respectively, and are formed on one surface of the substrate together with the band reject filter 19 so as to have a predetermined distance therebetween. As a result, since it is possible to reduce the capacitive coupling, thereby further reducing the radiation noise.

As described above, according to the wireless temperature sensor 100, a medical staff can collectively collect the patient's temperature data at the medical examination center without visiting the patient to measure the patient's temperature. Accordingly, since it is possible to considerably reduce the medical staff's efforts, it is possible to ensure time for other works.

In addition, according to the wireless temperature sensor 100, when the wireless communication is performed, a faint radio frequency or predetermined small power radio frequency in the range of 300 to 960 MHz band is used as a frequency of the carrier wave without requiring strict use examination. Accordingly, it is possible to enlarge a communication distance (for example, communication distance using a unit of 100 meters). As a result, since it is possible to use the entire area in the facilities as communicable area, it is possible to detect the patient's temperature variation at any place in the facilities. Furthermore, since a long-distance, communication is achieved with small energy, it is possible to reduce power consumption.

Moreover, in the wireless temperature sensor 100, the GND wire of the high frequency circuit 17 for driving the antenna is connected to the GND wire of a logic circuit of the wireless temperature sensor 100 through the band reject filter 19. Accordingly, the high frequency current of the carrier frequency band and of the vicinity of the carrier frequency band, which is generated by the logic circuit (digital circuit) of the wireless temperature sensor 100, is not input to the GND wire of the high frequency circuit 17 as a noise, thereby preventing the radiation noise from being generated.

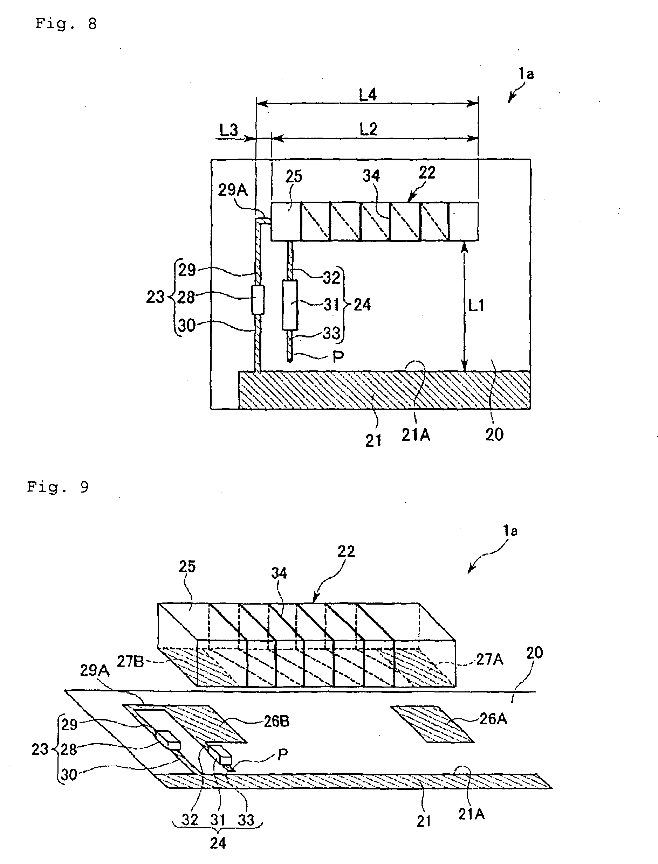

Next, an exemplary structure (chip antenna 1a) of the chip antenna 1 according to the embodiment of the invention will be described with reference to Figs. 8 and 9.

The chip antenna 1a is an antenna, which is used for a wireless device for mobile communication such as a mobile phone and a wireless device using a faint radio frequency or predetermined small power radio frequency.

As shown in Figs. 8 and 9, the chip antenna 1a includes an antenna substrate 20 made of an insulating material such as a resin, an earth part 21 that is a rectangular conductor film formed on the antenna substrate 20, a loading part 22, an inductor part 23, a capacitor part 24, and a feeding point P. The loading part, the inductor part, and the capacitor part are provided on one surface of the antenna substrate 20, and the feeding point P is connected to a high frequency circuit (not shown) provided outside the chip antenna 1a. Furthermore, an antenna operating frequency is adjusted by the loading part 22 and the inductor part 23 so that the chip antenna radiates an electric wave having a center frequency of 430 MHz.

The loading part 22 includes a spiral conductor pattern 34, which is formed on the surface of a rectangular parallelepiped element 25 in the longitudinal direction of the element. The rectangular parallelepiped element is made of, for example, a dielectric material such as alumina.

Both ends of the conductor pattern 34 are connected to connecting electrodes 27A and 27B formed on the lower surface of the element 25, respectively, so as to be electrically connected to rectangular mounting conductors 26A and 26B formed on the antenna substrate 20. In addition, one end of the conductor pattern 34 is electrically connected to the inductor part 23 and the capacitor part 24 through the mounting conductor 26B, and the other end thereof serves as an open end.

In this case, the loading part 22 is positioned away from the earth part 21 so that a distance L1 between the loading part and one side 21A of the earth part is, for example, 10 mm. Furthermore, the length L2 of the loading part 22 is, for example, 16 mm.

In addition, since a physical length of the loading part 22 is shorter than a quarter of an antenna operating wavelength, a self-resonant frequency of the loading part 22 becomes higher than the antenna operating frequency of 430 MHz. For this reason, when the antenna operating frequency of the chip antenna 1a is considered as a reference, it is not possible to conclude that the chip antenna self-resonates. Accordingly, the chip antenna is different from a helical antenna that self-resonates at an antenna operating frequency.

The inductor part 23 includes a chip inductor 28, and is connected to the mounting conductor 26B through an L-shaped pattern 29, which is a linear-conductive pattern formed on the antenna substrate 20. Moreover, the inductor part is connected to the earth part 21 through an earth part connecting pattern 30, which is a linear-conductive pattern formed on the antenna substrate 20 similar to the L-shaped pattern.

An inductance of the chip inductor 28 is adjusted so that the resonant frequency caused by the loading part 22 and the inductor part 23 is equal to 430 MHz of the operating frequency of the chip antenna 1a.

In addition, one side 29A of the L-shaped pattern 29 is parallel to the earth part 21, and a length L3 thereof is 2.5 mm. Accordingly, a physical length L4 of an antenna element parallel to one side 21A of the earth part 21 is 18.5 mm.

The capacitor part 24 includes a chip capacitor 31, and is connected to the mounting conductor 26B through a mounting conductor connecting pattern 32, which is a linear-conductive pattern formed on the antenna substrate 20. Moreover, the inductor part is connected to the feeding point P through a feeding point connecting pattern 33, which is a linear-conductive pattern formed on the antenna substrate 20 similar to the mounting conductor connecting pattern.

A capacitance of the chip capacitor 31 is adjusted so as to be match the impedance at the feeding point P.

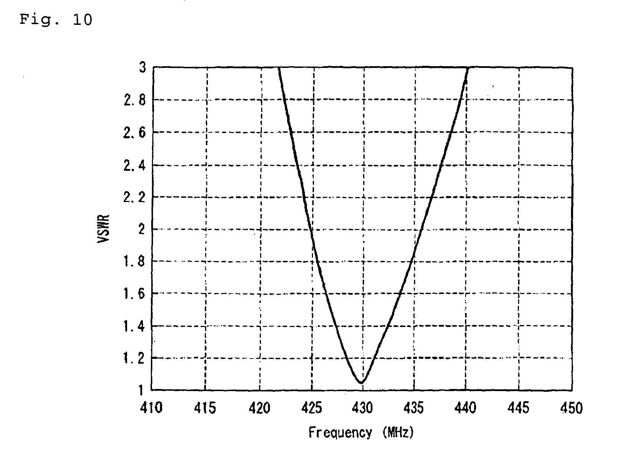

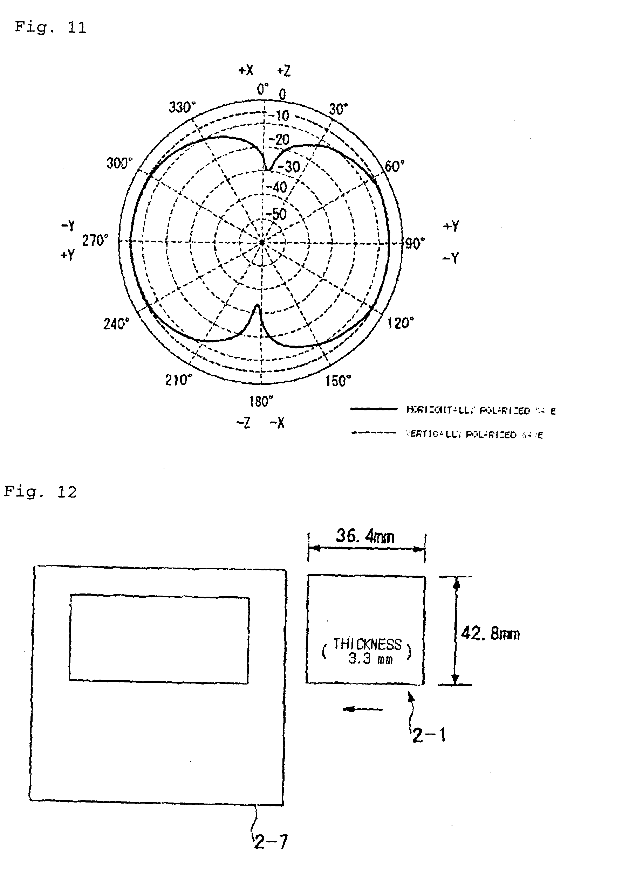

Figs. 10 and 11 show a frequency characteristic of VSWR (Voltage Standing Wave Ratio) at a frequency of 400 to 450 MHz, and the radiation pattern of a horizontally polarized wave and a vertically polarized wave, in the above-mentioned chip antenna 1a, respectively.

As shown in Fig. 10, the chip antenna 1a has a VSWR value of 1.05 at a frequency of 430 MHz, and has a bandwidth of 14.90 MHz at a VSWR value of 2.5.

When the chip antenna 1a according to the present embodiment is used, it is possible to reduce the length of the antenna so as to be shorter than an eighth of the frequency to be used, thereby considerably increasing a shortening coefficient.

### <Second aspect>

A second aspect of the invention will be described with reference to Figs. 12 to 20. A wireless interface device 2-1 according to a first embodiment will be described with reference to drawings. Fig. 12 is a block diagram showing a structure of the wireless interface device 2-1 according to the embodiment. In Fig. 12, a CF card (registered trade mark) is described as an example of the wireless interface device 2-1. The interface between the wireless interface device 2-1 and a PDA 7 is defined in accordance with interface standards of the wireless interface device 2-1 (I/O card edition standards of the CF card (registered trade mark) of a CFA; CompactFlash Association).

The wireless interface device 2-1 has, for example, a length of 42.8 mm, a width of 36.4 mm, and a thickness of 3.3 mm (these are dimension standards of the CF card (registered trade mark)). The wireless interface device is inserted into a socket of the PDA 2-7 for the CF card when used.

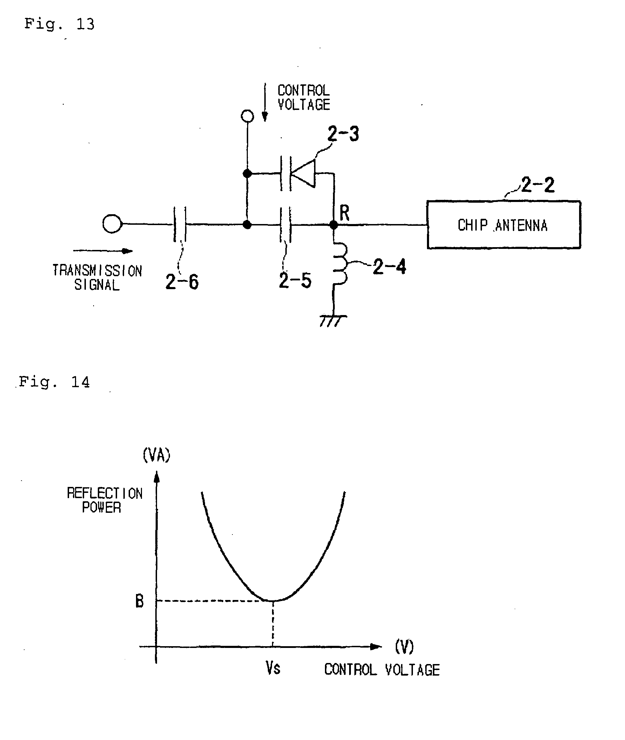

Accordingly, the size of an antenna provided to the wireless interface device 2-1 is limited to a length of 42.8 mm. For this reason, when a frequency between a faint radio frequency of 300 MHz band and a predetermined small power radio frequency of 900 or 960 MHz band is used as a frequency of the carrier wave to be used, the chip antenna 2 (see Fig. 13) needs to have the following length due to the fact that the length of an antenna is a quarter of a wavelength.

| Frequency | a quarter of a wavelength |
|---|---|
| 300 MHz | 250 mm |
| 400 MHz | 188 mm |
| 900 MHz | 83 mm |
| 960 MHz | 78 mm |

Therefore, in the present embodiment, shortening coefficients of 84% or more, 79% or more, 50% or more, and 55% or more are used to design / manufacture an antenna for frequencies of 300, 400, and 900 MHz bands, respectively, so that the chip antenna 2-2 is received in the wireless interface device 2-1 having a length of 42.8 mm.

In the chip antenna 2-2 used in the present embodiment, a resonant circuit for transmitting and receiving an electric wave is a composed of a resonant circuit, which includes an inductance component and a capacitance component, in order to configure an antenna having a high shortening coefficient and a small antenna having a high gain by using a circuit.

Furthermore, the chip antenna 2-2 of the wireless interface device 2-1 according to the present embodiment includes a plurality of resonant circuits to obtain a high gain. In addition, two or more resonant circuits, in which the inductance component and the capacitance component are electrically connected in parallel with each other, are electrically connected in series with each other. Moreover, the inductance component includes a coil, which is made of a conductor and is formed in a spiral shape or in the shape of a substantial spiral polygon about an axis thereof. The axis of the coil is provided at least in the resonant areas adjacent to each other in a shape of a substantial straight line, and at least one of parts of the conductor going around the axis is substantially included in the plane inclined with respect to the axis.

Accordingly, since a faint radio frequency or predetermined small power radio frequency in the range of 300 to 960 MHz band is used as a frequency of the carrier wave without requiring strict use examination, the wireless interface device 2-1 according to the present embodiment can enlarge a communication distance and reduce power consumption.

In addition, even though the wireless interface device 2-1 according to the present embodiment uses a frequency of the carrier wave having a long wavelength, the chip antenna 2-2 can be designed to be received in the wireless interface device 2-1. That is, shortening coefficients are determined and used to design the antenna so that the chip antenna 2-2 has a length and a width to be received in the wireless interface device 2-1 such as a memory card. Therefore, when the chip antenna is mounted in the wireless interface device, the wireless interface device 2-1 does not protrude from the PDA 2-7, thereby not spoiling the appearance design of the PDA 2-7.

However, since the chip antenna 2-2 includes the inductance component and the capacitance component, the chip antenna is easy to be affected by surroundings. That is, when the wireless interface device 2-1 is shipped, there is a case that the impedance of the chip antenna 2-2 matching with the impedance of a line for a transmission signal is varied by the influence of a metal case such as a PC or PDA 2-7, and the radiation characteristic thereof deteriorates. In order to remove the influence of the metal case, it is preferable that the chip antenna 1 be disposed away from the PC or PDA 2-7. However, this departs from the original object in which the chip antenna 2-2 having a large shortening coefficient is mounted in the wireless interface device 2-1.

Accordingly, the wireless interface device 2-1 according to the embodiment of the invention is provided with an impedance adjusting circuit shown in Fig. 13.

In the impedance adjusting circuit, a variable capacitance diode 2-3 and a capacitor 2-5 are connected in parallel with each other, and a DC blocking capacitor 2-6 is connected to one end of the parallel circuit of the variable capacitance diode and the capacitor. Then, a coil 2-4 is connected to the other end thereof. Furthermore, one end of the coil 2-4 is grounded. The chip antenna 2-2 is connected to a node R between the variable capacitance diode 2-3 and the capacitor 2-5.

When the transmission signal (a RF signal that is a traveling wave), which is obtained by modulating a high frequency carrier wave by using data, is reflected at a contact point (node R) having different impedance, the traveling wave is affected by a reflected wave. Accordingly, a composite wave, in which the traveling wave and the reflected wave are combined, is generated on the line. The composite wave is referred to as a standing wave, and a ratio between the maximum voltage | Vₘₐₓ | of the standing wave and the minimum voltage | Vₘᵢₙ | thereof is referred to as a voltage standing wave ratio (VSWR). When the reflection does not occur, the VSWR is 1. That is, as the value of the VSWR is reduced, the reflection is reduced. In case of a small antenna such as the chip antenna 2-2, a reference VSWR of the node R is about 3 or less.

A control unit (not shown) measures the VSWR of the node R, and applies control voltage to the matching circuit so that the VSWR is about 3 or less. The variable capacitance diode 2-3 has a characteristic in which the capacitance thereof is varied due to the applied control voltage (reverse voltage), thereby adjusting the impedance of the line. The relationship between the reflection power and the control voltage is shown in Fig. 14. A horizontal axis of Fig. 14 represents the control voltage (V), and a vertical axis thereof represents the reflection power (VA).

Therefore, according to the wireless interface device 2-1 of the present embodiment, in a state in which the wireless interface device 2-1 is mounted in the PDA 2-7 or the like, the control unit adjusts impedance between the chip antenna 2-2 and the line for the transmission signal. As a result, it is always possible to transmit the transmission signal so that transmission power has an almost maximum value. Accordingly, it is possible to prevent the radiation characteristic from deteriorating, and to improve receiver sensitivity.

When the wireless interface device 2-1 having the chip antenna 2-2 therein is inserted into the socket of the PDA 2-7 (or PC) for the wireless interface device 2-1 to be used, high frequency current caused by a digital signal of the PDA 2-7 is input to the wireless interface device 2-1 through the GND (ground) wire.

As a result, since a noise caused by the high frequency current is superimposed on the transmission signal, there is a possibility that the transmission wave having a noise component (radiation noise) is radiated from the chip antenna 2-2.

Specifically, if the frequency of the noise is in a carrier frequency band or in the vicinity of the carrier frequency band, the noise is radiated as a strong radiation noise. Accordingly, the noise has a negative effect on the reception characteristic of other wireless devices, which use the same carrier frequency band as the frequency of the noise.

However, since the radiation noise is different every device, it is difficult to remove the radiation noise from the receiving unit.

For this reason, the wireless interface device 2-1 according to the present embodiment modulates the carrier wave by using a modulation wave so as to generate the transmission signal. In addition, the GND wire of a high frequency circuit and the GND wire of an interface circuit are connected to each other through a band reject filter (or band elimination filter). In this case, a signal is radiated from the chip antenna 2-2 to the high frequency circuit, and the interface circuit transmits transmission data and a control signal to the PDA 2-7 (or PC) and receives transmission data and the control signal from the PDA 2-7 (or PC). Furthermore, the band reject filter blocks an only wave in a predetermined frequency band (a carrier frequency band at which the card is used and a frequency range including the vicinity of the carrier frequency band).

That is, the interface circuit is directly connected to the GND wire of the PDA 2-7 through a terminal of the socket. Meanwhile, the GND wire of the high frequency circuit is connected to the GND wire of the PDA 2-7 through the band reject filter. Accordingly, the high frequency current of the carrier frequency band and of the vicinity of the carrier frequency band, which is generated by the circuit of the PDA 2-7 and the interface circuit, is not input to the GND wire of the high frequency circuit as a noise, and it is possible to prevent the radiation noise from being generated.

Furthermore, the substrate provided in the wireless interface device 2-1 is configured so as to reduce the spatial coupling (capacitive coupling) between the GND wire of the interface circuit and the GND wire of the high frequency circuit. Accordingly, the GND wires are not formed on the upper and lower surfaces of the substrate, respectively, and are formed on one surface of the substrate together with the band reject filter so as to have a predetermined distance therebetween. As a result, since it is possible to reduce the capacitive coupling, thereby further reducing the radiation noise.

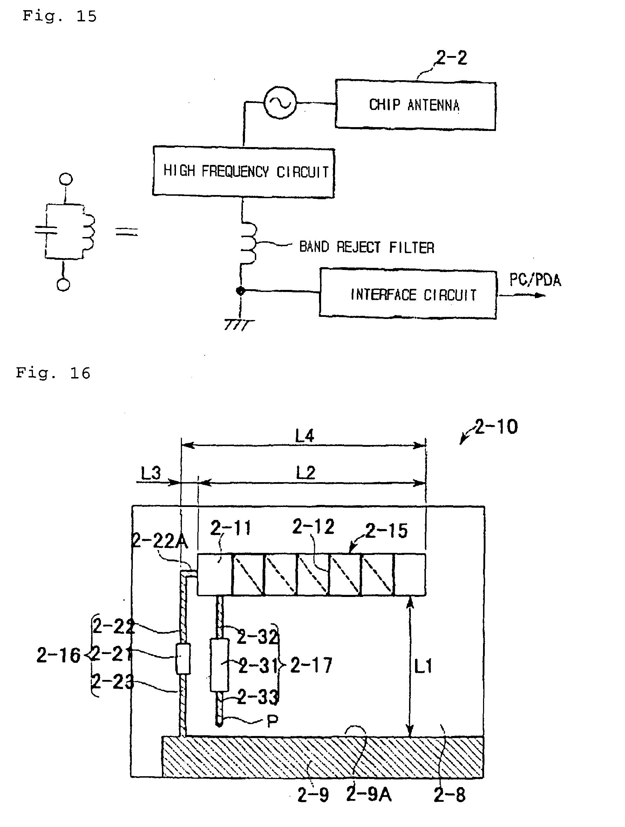

Moreover, according to the wireless interface device 2-1, as shown in Fig. 15, the GND wire of the high frequency circuit is connected to the GND wire of a logic circuit of the wireless interface device 2-1 and the GND wire of the PDA 2-7 (or PC) through the band reject filter. Accordingly, the high frequency current of the carrier frequency band and of the vicinity of the carrier frequency band, which is generated by the circuit of the PDA 2-7 and the logic circuit (interface circuit) of the wireless interface device 2-1, is not input to the GND wire of the high frequency circuit as a noise, thereby preventing the radiation noise from being generated.

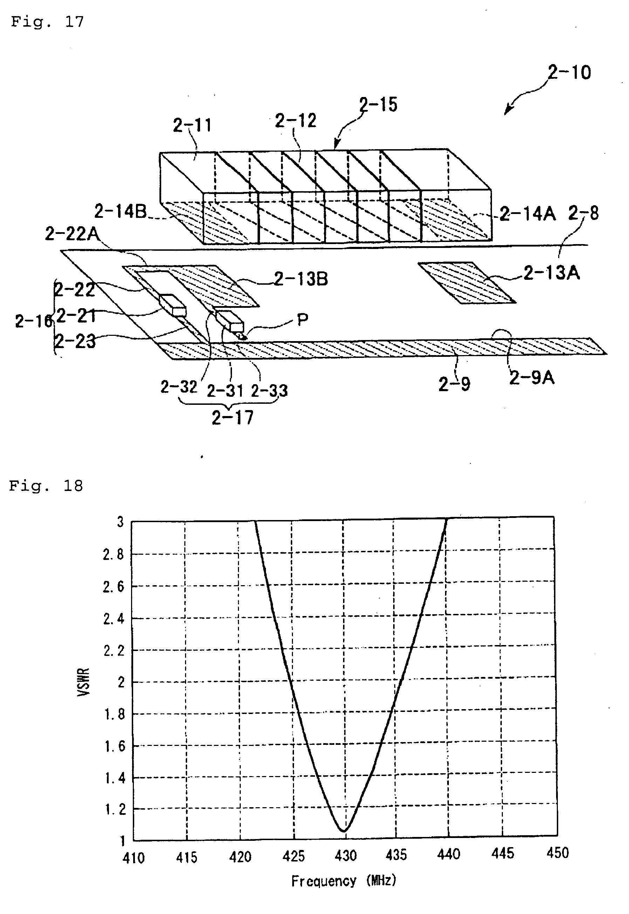

Next, a wireless interface device 2-10 according to a second embodiment of the invention will be described with reference to Figs. 16 and 17.

The wireless interface device 2-10 according to the present embodiment is a wireless interface device, which is used for a wireless device for mobile communication such as a mobile phone and a wireless device using a faint radio frequency or predetermined small power radio frequency.

As shown in Figs. 16 and 17, the wireless interface device 2-10 includes a substrate 2-8 made of an insulating material such as a resin, an earth part 2-9 that is a rectangular conductor film formed on the substrate 2-8, a loading part 2-5, an inductor part 2-16, a capacitor part 2-17, and a feeding point P. The loading part, the inductor part, and the capacitor part are provided on one surface of the substrate, and the feeding point is connected to a high frequency circuit (not shown) provided outside the wireless interface device 2-10. Furthermore, an antenna operating frequency is adjusted by the loading part 2-15 and the inductor part 2-16 so that the wireless interface device radiates an electric wave having a center frequency of 430 MHz.

The loading part 2-15 includes a spiral conductor pattern 2-12, which is formed on the surface of a rectangular parallelepiped element 2-11 in the longitudinal direction of the element. The rectangular parallelepiped element is made of, for example, a dielectric material such as an alumina.

Both ends of the conductor pattern 2-12 are connected to connecting electrodes 2-14A and 2-14B formed on the lower surface of the element 2-11, respectively, so as to be electrically connected to rectangular mounting conductors 2-13A and 13B formed on the substrate 2-8. In addition, one end of the conductor pattern 2-12 is electrically connected to the inductor part 2-16 and the capacitor part 2-17 through the mounting conductor 2-13B, and the other end thereof serves as an open end.

In this case, the loading part 2-15 is positioned away from the earth part 2-9 so that a distance L1 between the loading part and one side 9A of the earth part 2-9 is, for example, 10 mm. Furthermore, the length L2 of the loading part 2-15 is, for example, 16 mm.

In addition, since a physical length of the loading part 2-15 is shorter than a quarter of an antenna operating wavelength, a self-resonant frequency of the loading part 2-15 becomes higher than the antenna operating frequency of 430 MHz. For this reason, when the antenna operating frequency of the wireless interface device 2-10 is considered as a reference, it is not possible to conclude that the wireless interface device self-resonates. Accordingly, the wireless interface device is different from a helical antenna that self-resonates at an antenna operating frequency.

The inductor part 2-16 includes a chip inductor 2-21, and is connected to the mounting conductor 13B through an L-shaped pattern 2-22, which is a linear-conductive pattern formed on the substrate 2-8. Moreover, the inductor part is connected to the earth part 2-9 through an earth part connecting pattern 2-23, which is a linear-conductive pattern formed on the substrate 2-8 similar to the L-shaped pattern.

An inductance of the chip inductor 2-21 is adjusted so that the resonant frequency caused by the loading part 2-15 and the inductor part 2-16 is equal to 430 MHz of the operating frequency of the wireless interface device 2-10.

In addition, one side 22A of the L-shaped pattern 2-22 is parallel to the earth part 2-9, and a length L3 thereof is 2.5 mm. Accordingly, a physical length L4 of an antenna element parallel to one side 9A of the earth part 2-9 is 18.5 mm.

The capacitor part 2-17 includes a chip capacitor 2-31, and is connected to the mounting conductor 13B through a mounting conductor connecting pattern 2-32, which is a linear-conductive pattern formed on the substrate 2-8. Moreover, the inductor part is connected to the feeding point P through a feeding point connecting pattern 2-33, which is a linear-conductive pattern formed on the substrate 2-8 similar to the mounting conductor connecting pattern.

A capacitance of the chip capacitor 2-31 is adjusted so as to be matched with the impedance at the feeding point P.

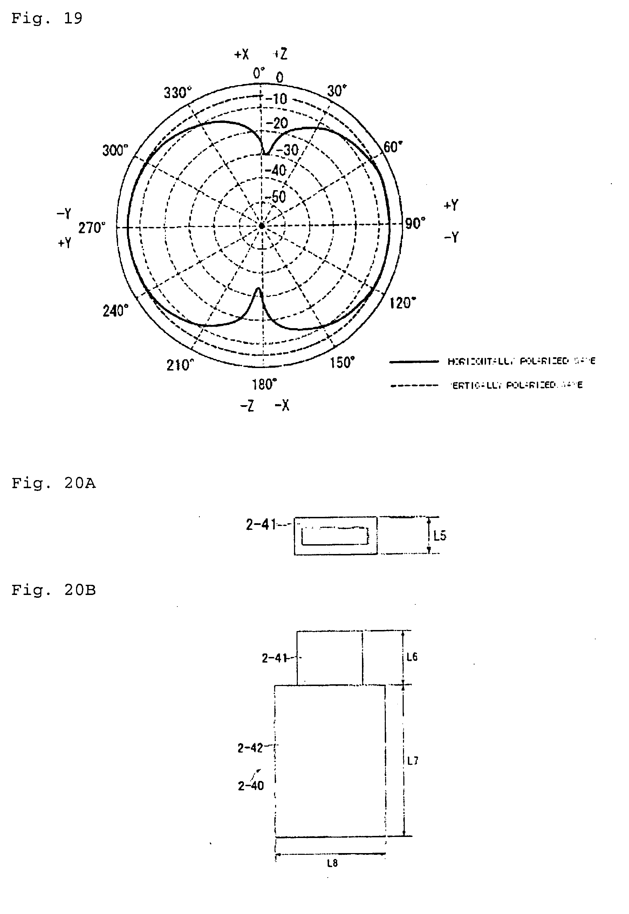

Figs. 18 and 19 show a frequency characteristic of VSWR (Voltage Standing Wave Ratio) at a frequency in the range of 400 to 450 MHz, and the radiation pattern of a horizontally polarized wave and a vertically polarized wave, in the above-mentioned wireless interface device 2-10, respectively.

As shown in Fig. 18, the wireless interface device 2-10 has a VSWR value of 1.05 at a frequency of 430 MHz, and has a bandwidth of 14.90 MHz at a VSWR value of 2.5.

When the wireless interface device 2-10 according to the present embodiment is used, it is possible to reduce the length of the antenna so as to be shorter than an eighth of the wavelength of frequency to be used, thereby considerably increasing a shortening coefficient. Accordingly, it is possible to mount the wireless interface device 2-10 in an USB (Universal Serial Bus) connector, which is a standard interface of a PC.

Figs. 20A to 20C show a shape of the USB connector having the wireless interface device 2-10 (Fig. 16) of the present embodiment mounted therein. The USB connector 2-40 is an USB connector corresponding to a series A plug. The USB connector 2-40 includes a PC connecting part 2-41 and a wireless communication part 2-42.

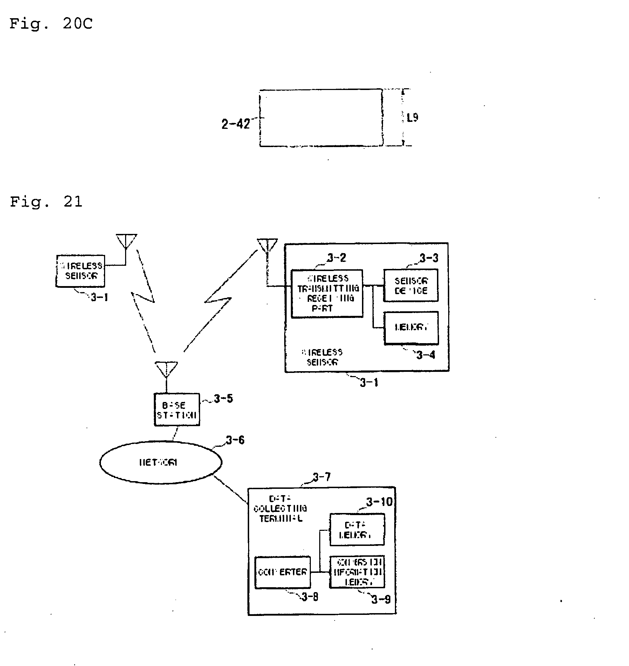

Fig. 20A is a front view of the PC connecting part 2-41, Fig. 20B is a plan view of the USB connector 2-40, and Fig. 20C is a rear view of the wireless communication part 2-42. Dimensions L5 to L9 of the USB connector 2-40 can be as follows: L5 = 7.5 mm, L6 = 12.0 mm, L7 = 30.0 mm, L8= 16.0 mm, and L9 = 10.0 mm.

Dimensions of the wireless interface device 2-10 shown in Fig. 16 is as follows: L1 = 10 mm and L4 = 18.5 mm. Since the wireless interface device is sufficiently smaller than the wireless communication part 2-42 of the USB connector 2-40 shown in Fig. 20, the wireless interface device 2-10 (Fig. 16) can be mounted in the wireless communication part 2-42 of the USB connector 2-40 shown in Fig. 20.

As described in each embodiment, each of the wireless interface devices (1 and 10) according to the above-mentioned first and second embodiments is provided with an antenna for transmitting and receiving an electric wave. In addition, each of the wireless interface devices (1 and 10) is provided with a transmitting / receiving circuit, which processes the signal received by the antenna, or an interface circuit, which interchanges data with the PDA 2-7 or PC, in addition to the antenna. As described above, each of the wireless interface devices (1 and 10) is provided with the antenna, the transmitting / receiving circuit, and the interface circuit. Accordingly, it is possible to perform the wireless communication by only inserting each wireless interface device (1 and 10) into the slot of the PDA 2-7 without adding special functions to the PDA 2-7.

Furthermore, the wireless interface devices (1 and 10) are separately described in the above-mentioned first and second embodiments, respectively. However, the invention is not limited thereto, and the both of the wireless interface devices can be combined to each other. For example, the structure of the antenna described with reference to Figs. 16 and 17 can be applied to the chip antenna 2-2 shown in Fig. 13 to configure a wireless interface device.

### <Third aspect>

A third aspect of the invention will be described with reference to Figs. 21 to 23.

Hereinafter, a wireless sensor system according to an aspect of the invention will be described with reference to the drawings. Fig. 21 is a block diagram showing an exemplary structure of the wireless sensor system. In Fig. 21, a plurality of wireless sensor 3-1 each include a wireless transmitting / receiving part 3-2, a sensor device 3-3, and a memory 3-4. The wireless transmitting / receiving part 3-2 wirelessly transmits measured results with identification numbers as measured data. The sensor device 3-3 measures physical quantities corresponding to environment information such as temperature, humidity, sound volume, and concentration of various gases, as measured values. The memory 3-4 stores a physical property, an initial value deviation, a kind of the sensor device 3-3, conversion information such as compensation information of the physical property, and an identification number of each wireless sensor. Here, the sensor device 3-3 measures temperature by using the resistance value (for example, a thermistor), which is a physical quantity as a measured value, and the sensor device 3-3 calculates temperature serving as environment information from the resistance value varied depending on the temperature with respect to the sensor temperature characteristic.

For this reason, the kind of the sensor device 3-3 represents the resistance value, and the physical property thereof represents a general formula (which may be a look up table showing the relationship between the temperature and the resistance value, and in this case, reads out the temperature corresponding to the resistance value) showing the relationship between the temperature and the resistance value. The initial value deviation is a deviation of the general formula, for example, at 25°C, and the compensation information of the physical property is a compensating rate (a slope deviation of a variation line, a curvature deviation of a variation curve, or the like) for the predetermined temperature to be obtained by the general formula.

Moreover, when the assembly process of the sensor is different from that of the sensor device 3-3, the compensation information of the physical property includes correction values of sensor peripheral circuits configuring the sensor device. Accordingly, the sensor and the sensor device 3-3 can be separately managed in the assembly process, thereby efficiently managing the assembly.

In addition, a correction coefficient for the secular change (annual change of the physical property) of the physical quantity, which is measured by the sensor device 3-3, is stored in the memory 3-4 as coefficient information in every elapsed period (for example, six months, one year, or the like). The correction coefficient is obtained as follows: the annual change of the sensor is obtained from the evaluation results of a plurality of similar sensors by an evaluation method such as aging, and the annual change is statistically processed to expect the correction coefficient.

A base station 3-5 receives data such as measured values wirelessly transmitted by each wireless sensor 3-1, and transmits the received data to a data-collecting terminal 3-7, which collects and analyzes the environment information, through a network 3-6. Here, the network 3-6 is an information network that includes private information communication lines, public information communication lines, and an Internet. The data-collecting terminal 3-7 includes a converter 3-8, a conversion information memory 3-9, and a data memory 3-10. The converter 3-8 converts the measured values into a numeral of the environment information by mean of the conversion information, the conversion information memory 3-9 stores the conversion information and correction coefficient of the sensor device 3-3 of each wireless sensor 3-1 in correspondence with the identification number, and the data memory 3-10 stores the numeral of the environment information of each wireless sensor 3-1 and a mounting position (measuring position) of each wireless sensor 3-1 for every identification number.

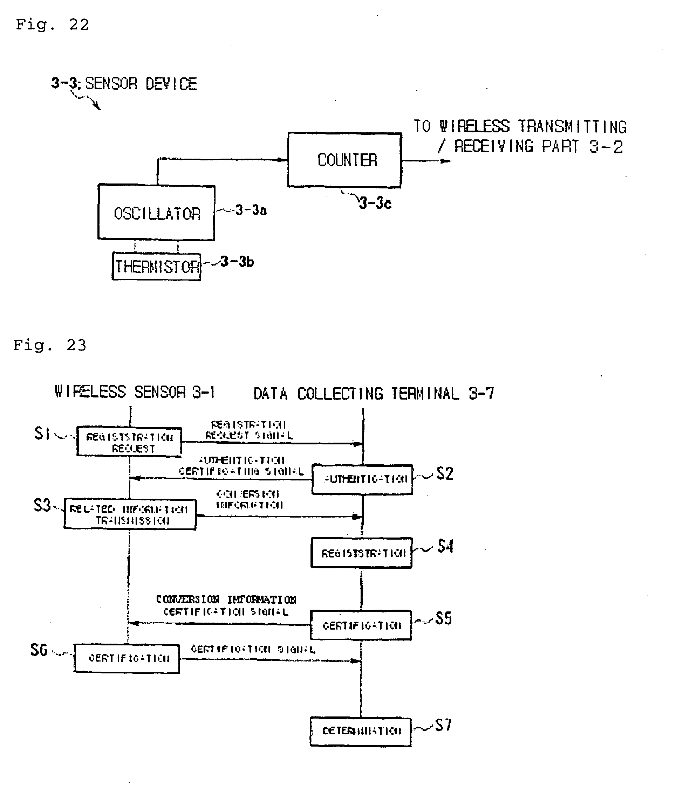

Next, the sensor device 3-3 of Fig. 21 will be described with reference to Fig. 22. Fig. 22 is a block diagram of a temperature sensor, which is an example of the sensor device 3-3 shown in Fig. 21. In Fig. 22, as the resistance value of the thermistor 3b is varied depending on the temperature variation, an oscillation frequency is varied in accordance with temperature. Accordingly, the oscillator 3a determines the oscillation frequency depending on the resistance value of a thermistor 3b. A counter 3c counts the number of pulses oscillated by the oscillator 3a, and outputs the counted values to the wireless transmitting / receiving part 3-2 in every predetermined period (for example, thirty minutes). Furthermore, after resetting the counted values to be 'zero', the counter counts new values during a predetermined period. That is, the sensor device 3-3 measures temperature, which is the environment information, as the number (oscillation frequency), of the pulses to be countered for the predetermined period, and outputs the counted number to the wireless transmitting / receiving part 3-2. The counted number of the pulses represents the resistance value (physical quantity) of the thermistor 3b (for example, a NTC (Negative Temperature Coefficient) thermistor or the like). A Wien bridge circuit stable against voltage variations and temperature variations is used in the oscillator 3a. For this reason, even though there are voltage variations of the power source, it is possible to obtain the resistance variations of the thermistor 3b depending on temperature variations by using the oscillation frequency. Therefore, it is possible to reliably measure temperatures.

Next, an exemplary operation of the wireless sensor system according to an embodiment will be described with reference to Fig. 21.

### <Registration process of wireless sensor 3-1 to data-collecting terminal 3-7>

An exemplary registration process of each wireless sensor 3-1 to the data-collecting terminal 3-7 in the wireless sensor system will be described with reference to Fig. 23. Fig. 23 is a sequence diagram showing the data transmission and reception, which is performed between each wireless sensor 3-1 and the data-collecting terminal 3-7 through the base station 3-5 and the network 3-6 therebetween. In the following description, the temperature sensor shown in Fig. 22 is used in the sensor device 3-3.

When the registration process of each wireless sensor 3-1 is performed, the wireless transmitting / receiving part 3-2 commences the registration process of each wireless sensor 3-1 to the data-collecting terminal 3-7 of each wireless sensor 3-1 by directing the wireless sensor 3-1 to perform the registration process to the wireless transmitting / receiving part 3-2, for example, by pushing a start button for the registration process.

The wireless transmitting / receiving part 3-2 transmits a registration request signal, which includes the address and identification number of each wireless sensor 3-1, to a predetermined data-collecting terminal 3-7 in accordance with a predetermined address that is preset in the memory 3-4 (step S1). When the data-collecting terminal 3-7 determines that the transmitted signal is a registration request signal, the data-collecting terminal 3-7 determines whether the identification number included therein is a registerable identification number preset in the signal data memory 3-10. Then, when the identification number included therein is the registerable identification number, the data-collecting terminal 3-7 transmits an authentication certificating signal for continuing the registration process to each wireless sensor 3-1 together with the identification number in accordance with the input address. Meanwhile, when the identification number included therein is not the registerable identification number, the data-collecting terminal 3-7 transmits an authentication certificating signal for stopping the registration process to each wireless sensor 3-1 so as to stop the registration process (step S2).

After that, when receiving the authentication certificating signal for continuing the registration process, the wireless transmitting / receiving part 3-2 reads out the conversion information and the correction coefficient stored in the memory 3-4. Then, the wireless transmitting / receiving part 3-2 transmits the read conversion information and correction coefficient to the data-collecting terminal 3-7 together with the identification number of each wireless sensor 3-1 (step S3). Here, the conversion information to be transmitted represents a relationship between the counted value (oscillation frequency) and the resistance value during the predetermined period when the temperature sensor (sensor device 3-3) of Fig. 22 outputs, a general formula between a resistance value (physical quantity) and temperature, a deviation of the general formula at 25°C, and a predetermined temperature compensating rate to be obtained from the general formula. The correction coefficient is a correction coefficient for the secular change of a physical quantity to be measured by the sensor device 3-3 in every elapsed period (for example, six months, one year, or the like).

Moreover, the data-collecting terminal 3-7 stores the transmitted conversion information and correction information in the conversion information memory 3-9 in correspondence with the identification number that is received simultaneously with the information (step S4).

Next, the data-collecting terminal 3-7 reads out the conversion information and correction information, which correspond to the registered identification number of each wireless sensor 3-1, from the conversion information memory 3-9. Then, the data-collecting terminal adds certificating requests to the conversion information and correction information, and transmits the information as a conversion information certificating signal to each wireless sensor 3-1 (step S5). When receiving the conversion information certificating signal, each wireless sensor 3-1 reads out the conversion information and correction information from the memory 3-4. Then, each wireless sensor compares the read conversion information and correction information having the added conversion information certificating signal, and determines whether they are the same or not. When it is determined that they are the same, the certificating signal for representing that they are the same is transmitted to the data-collecting unit 7. Meanwhile, when it is determined that they are not the same, the process returns to the step S3 (step S6). Next, when receiving the certificating signal for representing they are the same from the wireless sensor 3-1, the data-collecting terminal 3-7 detects that the conversion information and correction information are normally stored in the conversion information memory 3-9. Accordingly, the data-collecting terminal detects that each information is normally registered, and determines the registration (step S7).

### <Measurement of environment information>

Next, an exemplary operation of measuring the environment information in the wireless sensor system according to the embodiment will be described with reference to Figs. 21 and 22.

Each wireless sensor 3-1 adds an identification number to the counted value (measured value) output from the counter 3c in every predetermined period, for example, thirty minutes, and transmits the counted value having the identification number as a measured data to the data-collecting terminal 3-7 by means of the wireless transmitting / receiving part 3-2.

After that, when receiving the measured data, the data-collecting terminal 3-7 determines whether the identification number is stored in the conversion information memory 7. Then, when the identification number is detected from the conversion information memory 3-9, the data-collecting terminal 3-7 reads out the conversion information and correction information, which correspond to the identification number, by means of the converter 3-8.

Next, the converter 3-8 obtains the resistance value of the thermistor 3b from the relationship between the counted value (oscillation frequency) and the resistance value, on the basis of the read conversion information, for example, temperature. The converter 3-8 determines whether the elapsed period used to correct the correction information is passed. When the elapsed period is not passed, the resistance value is directly used. When the elapsed period is passed, the correction coefficient is multiplied by the resistance value, and then the product is used as a new resistance value. Next, the converter 3-8 includes a value of an initial value deviation as a compensating value in the general formula representing a relationship between the resistance value of the conversion information and temperature, and performs a calculation for obtaining temperature. Furthermore, the converter 3-8 multiplies the temperature, which is obtained from the general formula, by the correction coefficient set in every predetermined temperature, in order to perform a calculation for obtaining temperature as final environment information. Then, the converter allows the result of the calculation to corresponds to the identification number as temperature at the measured point, and allows the result thereof to be stored in the data memory 3-10 together with data of the measuring date and hour.

In addition, in case of temperature, the converter 3-8 may obtain temperature from the directly counted value by means of the general formula representing a relationship between a counted value and temperature. In this case, the converter determines whether the elapsed period used to correct the correction information is passed. When the elapsed period is not passed, the temperature is directly used. When the elapsed period is passed, the correction coefficient is multiplied by the temperature, and then the product is used as a new temperature.

Moreover, when transmitting the measured data, each wireless sensor 3-1 may measure an output voltage of the battery for supplying a driving electric power and may transmit the output voltage with the measured data. As a result, the data-collecting terminal 3-7 determines whether the output voltage is lower than a predetermined threshold value. When determining that the output voltage is larger than the threshold value, the data-collecting terminal continues the process. When determining that the output voltage is smaller than the threshold value, the data-collecting terminal allows the information for directing battery replacement to be displayed on the display.

In the above-mentioned wireless sensor 3-1 according to another embodiment, the wireless transmitting / receiving part 3-2 can be substituted for a wireless transmitting part 2B for only wirelessly transmitting an electric wave. In this case, at the time of registration, a registration request signal, which includes an address and an identification number of each wireless sensor 3-1, is transmitted to the data-collecting terminal 3-7 by a process similar to that of the embodiment. Then, the data-collecting unit 7 performs processes of authentication / registration in accordance with the identification number. For this reason, since it is unnecessary to have a receiving function, it is possible to reduce the size and power consumption of the wireless sensor. The other functions of the wireless sensor according to another embodiment are the same as those of the embodiment.

In addition, a program, which is used to realize function of the data-collecting terminal 7 shown in Fig. 21, may be recorded on the computer-readable recording medium, and the program recorded on the recording medium may be read out and executed by a computer system in order to collect the environment information. Furthermore, the above-mentioned 'computer system' includes an OS (operation system) and hardware such as peripheral devices. Moreover, the 'computer system' includes a WWW (World Wide Web) system having a homepage providing environment (or display environment). The 'computer-readable recording medium' is a portable medium such as a flexible disk, magneto-optical disc, ROM or CD-ROM, or a storage device such as a hard disk arranged in a computer system. Moreover, the 'computer-readable recording medium' includes a recording medium that holds a program for a predetermined time, such as a volatile memory (RAM) within a computer system, which serves as a server or client when the program is transmitted through a network such as the Internet or a communication channel such as a telephone line.

The program may be transmitted from a computer system having the program stored in the storage device thereof or the like to another computer system through a transmission medium or through transmission waves in the transmission medium. In this case, the 'transmission medium' for transmitting the program is a medium having a function of transmitting information, such as a network (communication network) like the Internet or a communication channel (communication line) like a telephone line. The program may be for realizing a part of the above-mentioned functions. The program may be a program that can realize the above-mentioned functions in combination with a program that is already recorded in a computer system, that is, a differential file (differential program).

As described above, embodiments of the invention have been described with reference to drawings. However, the specific structure of the invention is not limited to the embodiments, and the invention has various modifications and variations within the scope of the invention.

It is possible to mount the antenna in or on the device or substrate, which have limited dimensions, such as an IC card corresponding to the standard of a PCMCIA (Personal Computer Memory Card International Association) as well as a memory.

According to the wireless temperature sensor of the invention, even though a frequency of the carrier wave having a long wavelength is used, the length of the antenna is designed so that the antenna is received in the container. Accordingly, it is possible to make an antenna (chip antenna) small and to seal the wireless temperature sensor in the small container, which allows a patient to move freely.

In addition, according to the wireless temperature sensor of the invention, in a state in which the container is attached to a measuring object, it is possible to adjust the impedance matching between the antenna and a line for transmission signal. Accordingly, it is always possible to transmit the transmission signal so that transmission power has an almost maximum value, thereby preventing the radiation characteristic from deteriorating.

Furthermore, according to the wireless temperature sensor of the invention, the substrate is positioned in the container so that the antenna is separated from a contact surface to be attached to a measuring object with a predetermined distance therebetween. Accordingly, it is possible to reduce the capacitive coupling between the antenna and the measuring object, thereby reducing a coupling loss. Moreover, it is possible to suppress the deviation of the impedance between the antenna and the line for transmitting a transmission signal to the antenna, thereby performing a transmission with high efficiency.

In addition, according to the wireless temperature sensor of the invention, the wireless temperature sensor is formed in the shape of a 500-yen coin, that is, a coin having a diameter of 9 to 27 mm and a thickness of 5 to 10 mm. Accordingly, since the wireless temperature sensor can be attached to the skin and is easily carried without the common patient's discomfort, the wireless temperature sensor can be more widely used to measure temperature.

Moreover, according to the wireless temperature sensor of the invention, it is possible to reduce the length of the antenna so as to be shorter than an eighth of the wavelength of an carrier wave to be used, thereby considerably increasing the shortening coefficient of the antenna.

According to the wireless interface device of the invention, even though a frequency of the carrier wave having a long wavelength is used, the length of the antenna is designed with a shortening coefficient that allows the antenna be received in the wireless interface device. Accordingly, the antenna does not protrude from the device, and the appearance design of a small mobile terminal such as a PDA interface does not deteriorate.

In addition, according to the wireless interface device of the invention, in a state in which the wireless interface device is inserted into the socket of a PDA or the like so as to be mounted in the PDA or the like, it is possible to adjust the impedance matching between the antenna and a line for transmission signal. Accordingly, it is always possible to transmit the transmission signal so that transmission power has an almost maximum value, thereby preventing the radiation characteristic from deteriorating.

Furthermore, according to the wireless interface device of the invention, a grounding point of a high frequency circuit is connected to a grounding point of a logic circuit through a filter for blocking a signal of a carrier frequency band used for communication. Accordingly, the high frequency current of the carrier frequency band and of the vicinity of the carrier frequency band, which is generated by the circuit of the PDA and the logic circuit of the wireless interface device, is not input to the GND wire of the high frequency circuit as a noise, thereby preventing the radiation noise from being generated.

Moreover, according to the wireless interface device of the invention, it is possible to reduce the length of the antenna so as to be shorter than an eighth of the frequency of an carrier wave to be used, thereby considerably increasing a shortening coefficient of the antenna. Accordingly, it is possible to mount the wireless interface device in the USB connector.

According to the wireless sensor system of the invention, the wireless sensors, which are mounted at several measuring positions to measure environment information, does not calculate numerals of the environment information from the physical quantities, that is, measured values measured by the sensor devices. Accordingly, the structure of each wireless sensor can be simple and for general use. As a result, it is possible to mass-produce the wireless sensors, thereby reducing the manufacturing cost thereof.

In addition, according to the wireless sensor system of the invention, since the data-collecting terminal calculates the environment information from the measured values transmitted from the wireless sensors, it is possible to cope with several kinds of environment information. As a result, it is possible to configure a system corresponding to various sensor devices, thereby improving accuracy of environmental analysis at measuring positions by the several kinds of environment information.

Furthermore, according to the wireless sensor system of the invention, physical properties and conversion information of the sensor device in each wireless sensor are registered in the conversion information memory in correspondence with the identification number of each wireless sensor at the time of registration. Then, the environment information is calculated from the measured values on the basis of the information stored in the conversion information unit. Accordingly, it is possible to easily add wireless sensors to the system.

In addition, according to the wireless sensor system of the invention, a correction coefficient, which is used to correct the measured values at a predetermined time cycle, is registered in the conversion information memory at the time of registration. Then, the measured values are corrected by using the correction coefficient, and correction information is also stored in the conversion information memory. Accordingly, it is possible to easily correct converted numerals of the environment information, thereby obtaining accurate environment information.

## Claims

1. A wireless module comprising:
an antenna that is provided in a module main body and has a wireless communication function,
wherein the antenna has a length of a shortening coefficient so as to be received in the main body.

2. A wireless temperature sensor that is sealed in a container of a sensor main body and has a function of wirelessly transmitting measured data through an antenna,
wherein the antenna has a length of a shortening coefficient so as to be received in the container, and is received in the container.

3. The wireless temperature sensor according to claim 2, further comprising:
a matching circuit for adjusting the impedance between the antenna and a line for a transmission signal.

4. The wireless temperature sensor according to claim 3,
wherein the antenna and an electric circuit including the matching circuit are formed on one substrate, and the antenna is mounted on the substrate in an area where a grounding wire is not formed.

5. The wireless temperature sensor according to claim 4,
wherein the substrate is positioned in the container so that the substrate is separated from a contact surface to be attached to a measuring object with a predetermined distance therebetween.

6. The wireless temperature sensor according to claim 4 or 5,
wherein, on the substrate, a grounding point of a high frequency circuit is connected to a grounding point of a logic circuit through a filter for blocking a signal of a carrier frequency band used for communication.

7. The wireless temperature sensor according to any one of claims 2 to 6,
wherein the container has the same size as a 500-yen coin.

8. The wireless temperature sensor according to any one of claims 2 to 6,
wherein the container is formed in the shape of a coin that has a diameter of 9 to 27 mm and a thickness of 5 to 10 mm.

9. The wireless temperature sensor according to according to any one of claims 2 to 8,
wherein a length of the antenna is shorter than an eighth of the wavelength of an electric wave having a frequency to be used.

10. The wireless temperature sensor according to claim 9,
wherein the frequency to be used is in the range of 300 to 960 MHz.

11. The wireless temperature sensor according to any one of claims 2 to 10,
wherein the antenna includes an antenna substrate, a conductor film formed on a part of the antenna substrate, a feeding point formed on the antenna substrate, a loading part that is mounted on the antenna substrate and includes a linear-conductor pattern formed on the surface of an element made of a dielectric material in the longitudinal direction of the element, an inductor part for connecting one end of the conductor pattern with the conductor film, and a feeding point used to feed electric power to a node between the one end of the conductor pattern and the inductor part, and
the antenna is mounted on the substrate so that the longitudinal direction of the loading part is parallel to one side of the conductor film.

12. A wireless interface device that corresponds to an interface of a memory and includes an antenna having a wireless communication function,
wherein the antenna, which is used to transmit and receive a carrier wave, has a length of a shortening coefficient so as to be received in the memory having a standard dimension, and
the antenna is mounted in the wireless interface device.

13. The wireless interface device according to claim 12, further comprising:
a matching circuit for adjusting the impedance between the antenna and a line for a transmission signal.

14. The wireless interface device according to claim 12 or 13,
wherein a grounding point of a high frequency circuit is connected to a grounding point of a logic circuit through a filter for blocking a signal of a carrier frequency band used for communication.

15. The wireless interface device according to claim 12,
wherein the antenna includes a substrate, a conductor film formed on a part of the substrate, a feeding point formed on the substrate, a loading part that is mounted on the substrate and includes a linear-conductor pattern formed on the surface of an element made of a dielectric material in the longitudinal direction of the element, an inductor part for connecting one end of the conductor pattern with the conductor film, and a feeding point used to feed electric power to a node between the one end of the conductor pattern and the inductor part, and
the antenna is mounted in the wireless interface device so that the longitudinal direction of the loading part is parallel to one side of the conductor film.

16. The wireless temperature sensor according to claim 2, further comprising:
a plurality of wireless sensors that is provided at several positions, and that obtains measured values corresponding to surrounding environment information by means of a sensor device and transmits the values;
a data-collecting terminal that is provided to a base station, and that receives the measured values from the wireless sensors and calculates environment information from the measured values to collect the environment information of the several positions;
a sensor for outputting the surrounding environment information as measured values based on the physical property of the sensor device; and
a wireless transmitting part for wirelessly transmitting measured data including the measured values and identification data,
wherein the data-collecting terminal includes a conversion information memory, which stores conversion information used to convert a measured value of the sensor device of each registered wireless sensor into environment information, and a converter, which determines the wireless sensor by means of the identification data and converts the measured value into environment information by means of physical property and conversion information corresponding to each wireless sensor.

17. The wireless temperature sensor according to claim 2, further comprising:
a memory in which the physical property and conversion information of the sensor device is stored,
wherein, during the registration for the data-collecting terminal, the wireless terminal transmits the physical property and conversion information to the data-collecting terminal, and the data-collecting terminal stores the physical property and conversion information in the conversion information memory in correspondence with the identification number of the wireless sensor.

18. The wireless temperature sensor according to claim 2,
wherein the conversion information includes initial deviation of the physical property and compensation information of the physical property.

19. The wireless temperature sensor according to claim 2,
wherein the correction coefficient is stored in the sensor device used in the wireless sensor as data of the annual change of the physical property, which is statistically obtained, and
a time cycle of correction and the correction coefficient are transmitted to the data-collecting terminal at the time of registration.

20. The wireless temperature sensor according to claim 2,
wherein the wireless sensor measures an output voltage of a battery for supplying a driving electric power at a predetermined time cycle, and transmits information for directing battery replacement to the data-collecting terminal when the output voltage is smaller than a predetermined voltage.
